# EUROPEAN PATENT APPLICATION

(11) **EP 1 310 242 A1**
(43) Date of publication of application: **14.05.2003**
(21) Application number: 01830699.3
(22) Date of filing: 13.11.2001
(51) Int. Cl.: A61K 9/00, A61F 2/06

(54) **Carrier and kit for endoluminal delivery of active principles**

(71) Applicant: SORIN BIOMEDICA CARDIO S.p.A., 13040 Saluggia (Vercelli) (IT)
(72) Inventor: Vallana, Franco, 10123 Torino (IT); Curcio, Maria, 13040 Saluggia (Vercelli) (IT); Cassullo, Maria Cristina, 13048 Santhià (Vercelli) (IT); Grignani, Andrea, 10023 Chieri (Torino) (IT); Bottelli, Andrea, 16154 Genova Sestri Ponente (IT)
(74) Representative: Bosotti, Luciano

(57) **Abstract**

A carrier for delivering at least one active principle at an intraluminal site with at least one first region (C) and at least one second region comprises a carrier body (2), such as a stent, which can be conveyed to said intraluminal site, for example by means of catheterization. The carrier body (2) is provided with at least one recess (4) which has the function of a reservoir for receiving the active principle or principles. Present in the receiving reservoir is a filling of nanoparticles (1) which convey at least one respective active principle and comprise a substance having characteristics of preferential affinity attraction in regard to a preferred region between said at least one first region and said at least one second region. Consequently, when the carrier is located at said intraluminal site, each of said nanoparticles mainly migrates towards the preferred region in regard to which the aforesaid substance has characteristics of preferential affinity attraction.

## Description

The present invention relates to intraluminal delivery of active principles or agents.

The invention has been developed with particular attention paid to its possible application to stents, a subject to which a very extensive literature has been devoted. This also applies at a patent level, as is witnessed, for example, by the documents EP-A-0 806 190, EP-A-0 850 604, EP-A-0 857 470, EP-A-0 875 215, EP-A-0 895 759, EP-A-0 895 760, EP-A-1 080 738, EP-A-1 088 528, and EP-A-1 103 234, all of which are assigned to the Assignee of present application.

In particular, the present invention belongs in the line of research aimed at developing solutions that enable active or activatable agents of various kinds to be transported on a stent (or on a carrier of a different nature). When stents are used, the agents may be, for example, pharmacological agents, radioactive agents, etc., designed, for instance, to perform an antagonistic function in regard to restenosis.

Solutions of the above kind are described, within the above-cited documents, in EP-A-0 850 604, EP-A-1 080 738, and EP-A-1 103 234.

EP-A-0 850 604 describes the possibility of providing, on the surface of a stent, and in particular on its outer surface, a sculpturing having the function of increasing the surface area of the stent in such a way as to create undercuts and/or, in general, a surface roughness in order to facilitate application of coatings of active or activatable agents. The said sculpturing, consisting for instance of microspheres, may also favour adhesion of the stent to the wall of the vessel being treated.

Again the document EP-A-0 850 604 envisages the possibility of bestowing on the sculpture in question the aspect of grooves, channels, hollow parts or recesses designed to receive the aforesaid active principles or agents (the latter two terms being used as completely equivalent to one another in the context of the present description).

A solution of the above type is taken up also in WO-A-98 23228, EP-A-0 950 386, and again in the European patent application 01830489.9.

The solution described in the latter patent application, included in the state of the art under the provisions of Article 54(3) CBE, envisages that in the elements of the reticular structure of the stent there are provided recesses that are designed to perform the function of actual reservoirs for receiving agents for treatment of the site of implantation of the stent. Where present, the recesses confer on the respective element a hollowed sectional profile, of which the recesses occupy a substantial portion. The geometry of said recesses is chosen in such a way as to leave unimpaired the characteristics of bending strength of the respective element.

The above solution enables the amount of agent associated with the stent to be in any case sufficient, even when the aim is to obtain a release, and hence an action, that is prolonged in time. To the above there is added the consideration that, in applications of vascular angioplasty, the surfaces of the stent, and above all the inner surface, are subjected to an action of flushing by the blood flow.

Furthermore, the above solution enables the active or activatable agent to be made available and released prevalently, if not exclusively, on the outer surface of the stent, and not, instead, on its inner surface. This is true above all in the case where the agent applied on the stent is designed to perform an antagonistic function in regard to restenosis. The corresponding mechanism of action, which is aimed at acting on the outer surface of the stent facing the wall of the vessel that is undergoing treatment, may in fact have unfavourable effects in areas corresponding to the inner surface; for example, phenomena of neointimal formation on the inner surface of the stent, which are considered to be undoubtedly beneficial in the phases subsequent to the implantation phase, may prove hindered.

The solution in question thus makes it possible to have available stents that are able to take on the configuration of actual carriers of active or activatable agents, possibly different from one another, which are made available in sufficient quantities to achieve a beneficial effect that may also be prolonged over time, together with the further possibility of making available agents that are even different from one another and are selectively located in different positions along the development of the stent, in such a way as to enable selective variation of the dosages in a localized way, for instance achieving dosages that are differentiated in the various regions of the stent.

The solutions described above hence primarily meets requirements linked to the mechanism of release of the active agent. This applies in particular as regards i) the amount of agent that can be released; ii) the position in which the agent (or the various agents) arranged on the stent is (are) released; and, although to a lesser extent, iii) the time law of delivery/release of the active agent.

Another one of the documents referred to in the introductory part of the present description, namely EP-A-1 080 738, envisages associating, to the structure of an angioplasty stent, fibres constituting carriers for cores of restenosis-antagonistic agents. In a preferred way, the aforesaid cores are at least in part incorporated in nanoparticles, which are associated to the aforesaid fibres and are provided with an envelope made of bio-erodible material.

By "nanoparticles" is meant in general corpuscles having a spherical or substantially spherical shape and diametral dimensions typically of an order up to hundreds of nanometres. The nanoparticles in question may present an altogether homogeneous structure, i.e., a so-called "monolithic" structure, formed for example by a substantially homogeneous dispersion of a particulate substance in a mass having the function of a matrix, or else may have a core surrounded by an outer envelope. The core and the envelope may have a non-unitary structure, namely, a multiple structure (for example, with the presence of a number of cores or subcores) and/or a stratified structure, even with different formulations from one element to another.

For a more general illustration of the characteristics of the aforesaid nanoparticles, useful reference may be made to the works listed below.
Arshady R; Microspheres and microcapsules: a survey of manufacturing techniques. 1: Suspension and crosslinking. Polym. Eng. Sci. 1989; 30(15): 1746-1758.
Arshady R; Microspheres and microcapsules: a survey of manufacturing techniques. 3: Solvent evaporation. Polym. Eng. Sci. 1989; 30(15): 915-924.
Ruxandra G, *et al*.; Biodegradable long-circulating polymeric nanoparticles. Science 1994; 263: 1600-1603.
Kreuter J; Evaluation of nanoparticles as drug-delivery systems. I - Preparation method. Pharm. Acta Helv. 1983; 58(7): 196-209.
Narayani R, *et al.*; Controlled release of anticancer drug methotrexate from biodegradable gelatin microspheres. J. Microencapsulation. 1994; 11(1): 69-77.
Guzman LA, *et al.*; Local intraluminal infusion of biodegradable polymeric nanoparticles. Circulation 1996; 94: 1441-1448.
Jeyanthi R, *et al.*; Preparation of gelatin microspheres of bleomycin. International Journal of Pharmaceutics. 1987; 35: 177-179.
Pellizzaro C, *et al*.; Cholesteryl Butyrate in solid lipid nanospheres as an alternative approach for butyric acid delivery. Anticancer Research. 1999; 19: 3921-3926.
Cavalli R, *et al.*; Preparation and characterization of solid lipid nanospheres containing paclitaxel. European Journal of Pharmaceutical Sciences. 2000; 10: 305-309.

In particular, in EP-A-1 080 738 the use is envisaged of nanoparticles of the type comprising at least one core surrounded by an envelope which possibly has a stratified structure. The core comprises an agent that is able to perform an antagonistic function in regard to restenosis as a result of an action of localized release and/or penetration into the wall of the vessel that has undergone stent implantation. The core (or cores) in question may consist, for example, of a drug or a complex of drugs which are provided with an anti-inflammatory action, an anti-mitotic action and/or an action that promotes processes of repair of the wall of the vessel and which are able to mitigate or prevent the reactions that lie at the basis of the restenosis process.

The outer envelope of the nanoparticles consists, instead, of any substance that may be defined as "bio-erodible", i.e., able to be worn away and/or to assume or present a porous morphology, or in any case a morphology such as to enable diffusion outwards of the substance or substances included in the core. The characteristics of bio-erodibility are typically accompanied by characteristics of biocompatibility and biodegradability.

The substances that can be used for making the envelopes of the nanoparticles according to the aforesaid prior document are, for example, polyethylene glycol (PEG) and polylactic-polyglycolic acid (PLGA).

The solution proposed in EP-A-1 080 738 thus makes it possible to configure the stent as a carrier which, once it is placed in an intraluminal position, is able to perform the function of a true release machine, for controlled delivery of restenosis-antagonistic agents. This applies above all as regards the possibility of a precise control of the release kinetics, with the added possibility of selectively controlling release of different agents over time.

Also the solution proposed in EP-A-1 080 738 thus mainly acts on the mechanism of release of the active agents that can be associated to the stent or to any other type of carrier that can be placed in an intraluminal position.

The present invention tackles a problem which is, to a certain extent, complementary to the problem faced by the solutions according to the prior art, namely, that of controlling the kinetics of release of the active agents also as regards control of the interaction with the intraluminal site in which the carrier is placed, namely, in the case of stents (an example to which reference will continue to be made in the remaining part of the present description), the part of the vessel in which the stent is implanted and the surrounding regions.

According to the present invention, the above problem is tackled and solved thanks to a carrier for intraluminal delivery of active agents which has the characteristics specifically referred to in the claims which follow. The invention also relates to the corresponding kit, comprising a carrier of the above-specified type combined with an inserter means for placing the carrier in an intraluminal site. Preferably, the inserter means is a catheter, and, even more preferably, a balloon catheter.

Substantially, the solution according to the invention is largely based upon the composition of the nanoparticles, and preferably upon the composition of the envelope and/or upon its thickness, both with a view to obtaining a more or less fast release of the active principle contained therein and with a view to enabling the nanoparticles and agents contained in the envelopes to be selectively "guided" towards given areas or regions, more especially towards particular types of tissue of the environment surrounding the carrier, thus achieving a sort of selective attraction of the active principles by the areas (tissues, organs, etc.) that function as targets. In other words, the nanoparticles are provided with a sort of force of attraction that guides them in the direction of the target. The invention thus creates a release system that has a very high degree of efficiency, with the consequent possibility of reducing the absolute amount of active agent or principle that is to be administered.

In the above connection it will be noted that - as, on the other hand, is evident to a person skilled in the sector - although the present invention has been developed with particular attention paid to its possible application to stents, its scope is altogether general, and consequently the invention may be applied to any type of carrier that is designed to be placed in an intraluminal position (i.e., inside any vessel of the human body), for example by means of catheterization.

The present invention will now be described, purely by way of non-limiting example, with reference to the attached drawings, in which:
- Figure 1 is a schematic illustration of the characteristics of nanoparticles that can be used in the framework of the invention;
- Figure 2 illustrates, in general terms, the operating principle of the invention applied to an angioplasty stent; and
- Figures 3 to 9 illustrate as many different modes of use of the invention, again applied to an angioplasty stent.

By way of an introduction to the detailed description of some examples of embodiment of the invention, it is once again recalled that, although the present invention has been developed with specific attention paid to its possible application to stents, in particular to angioplasty stents, its range of application is altogether general. The solution according to the invention can be applied to any carrier which can be placed, for example by means of catheterization, in an intraluminal position, i.e., inside a vessel of the human body or of the body of an animal which is to undergo a type of treatment that involves, as a main step or as an accessory step, delivery of an active principle or agent, for instance in the form of a drug.

On the basis of the above introductory remark it will therefore be understood that the invention can be applied, for example (and without the possibility of the ensuing list being considered in any way limiting), in addition to stents, such as angioplasty stents, to vascular grafts, to the so-called stents/grafts, to catheters for percutaneous coronary balloon angioplasty (PTCA) treatments, catheters for mechanical/electrical ablation of endovascular plaques, catheters or electrodes for the elimination or passivation (again by mechanical, electrical and/or chemical means) of the so-called ectopic foci responsible for fibrillation phenomena, electrodes for electrostimulation /defibrillation, electrodes for endocardial mapping, endoscopes and similar devices.

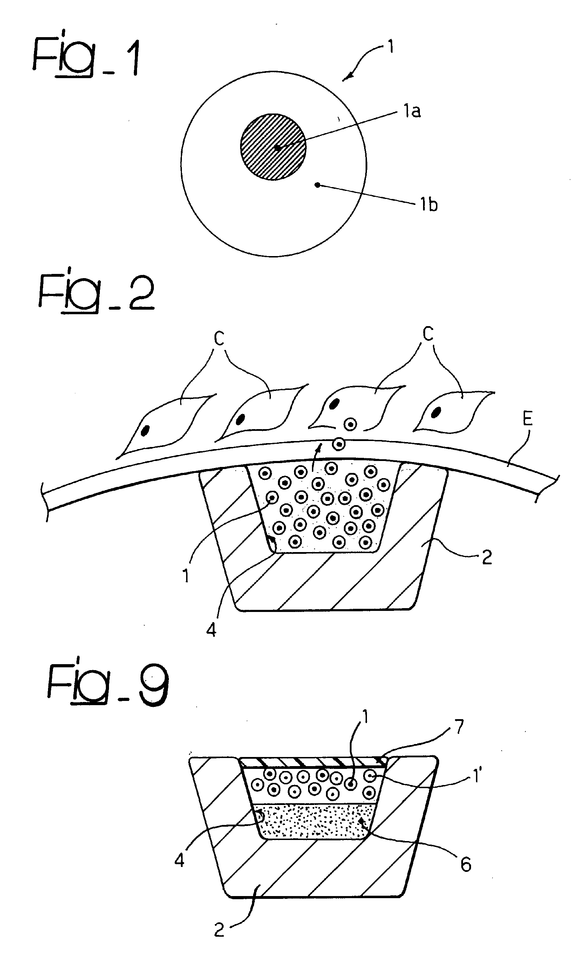

Figure 1 illustrates the characteristics of a structure 1 of the type currently referred to as "nanoparticle".

By this name are generally meant (see in this connection the references quoted in the introductory part of the present description) corpuscles having a spherical or substantially spherical shape and diametral dimensions typically of the order of hundreds of nanometres.

In the example of embodiment of the invention herein illustrated (which, it is recalled, is merely an example), the nanoparticles 1 usually comprise a core 1a surrounded by an outer envelope 1b.

Figure 1 shows that the core 1a, instead of being in a substantially central position, may be in an eccentric position with respect to the envelope 1b. Again, whilst Figure 1 shows a nanoparticle comprising a single core 1a, it is possible to obtain nanoparticles 1 that have a multiple structure (for example, with the presence of a number of cores or subcores). And again, whilst Figure 1 of the annexed drawings shows an envelope 1b with a substantially uniform structure, it is possible to obtain envelopes 1b having a stratified structure.

The core 1a may be made or may comprise any agent (the term being used herein in its widest sense, and hence such as to comprise any active/activatable principle or any drug) which is able to perform an action, in particular a local action, on the site where the corresponding carrier (illustrated in greater detail in what follows) is placed in an intraluminal position.

To clarify the concept and with reference (without this being viewed in any way as limiting the scope of the invention), the agent or agents that make up the core or cores 1a of the nanoparticles 1 or that are comprised therein may consist of a drug or a complex of drugs with an anti-inflammatory action, such as the ones listed below.

| Corticosteroids: | | |
|---|---|---|
| Cortisol | Betamethasone | Fluocinolone |
| Cortisone | Dexamethasone | Fluocinonide |
| Corticosterol | Flunisolide | Fluorometholone |
| Tetrahydrocortisol | Alclomethasone | Fluorandrenolide |
| Prednisone | Amcinonide | Alcinonide |
| Prednisolone | Clobetasol | Medrisone |
| Methylprednisolone | Clocortolone | Momethasone |
| Fluodrocortisone | Desonide | Rofleponide |
| Triamcinolone | Desoxymethasone | |
| Paramethasone | Diflorasone | |

as well as all the corresponding esters, salts and derivatives.

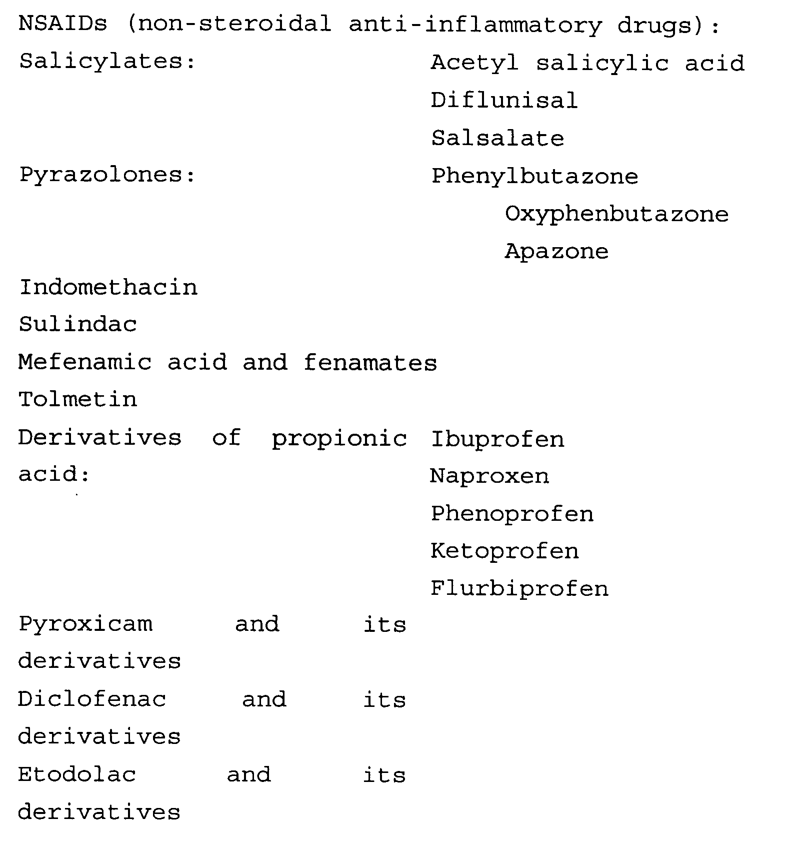

In addition or as an alternative, the active agent or principle may comprise a drug or a complex of drugs with antineoplastic action, such as the ones listed below.

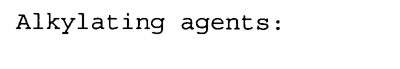

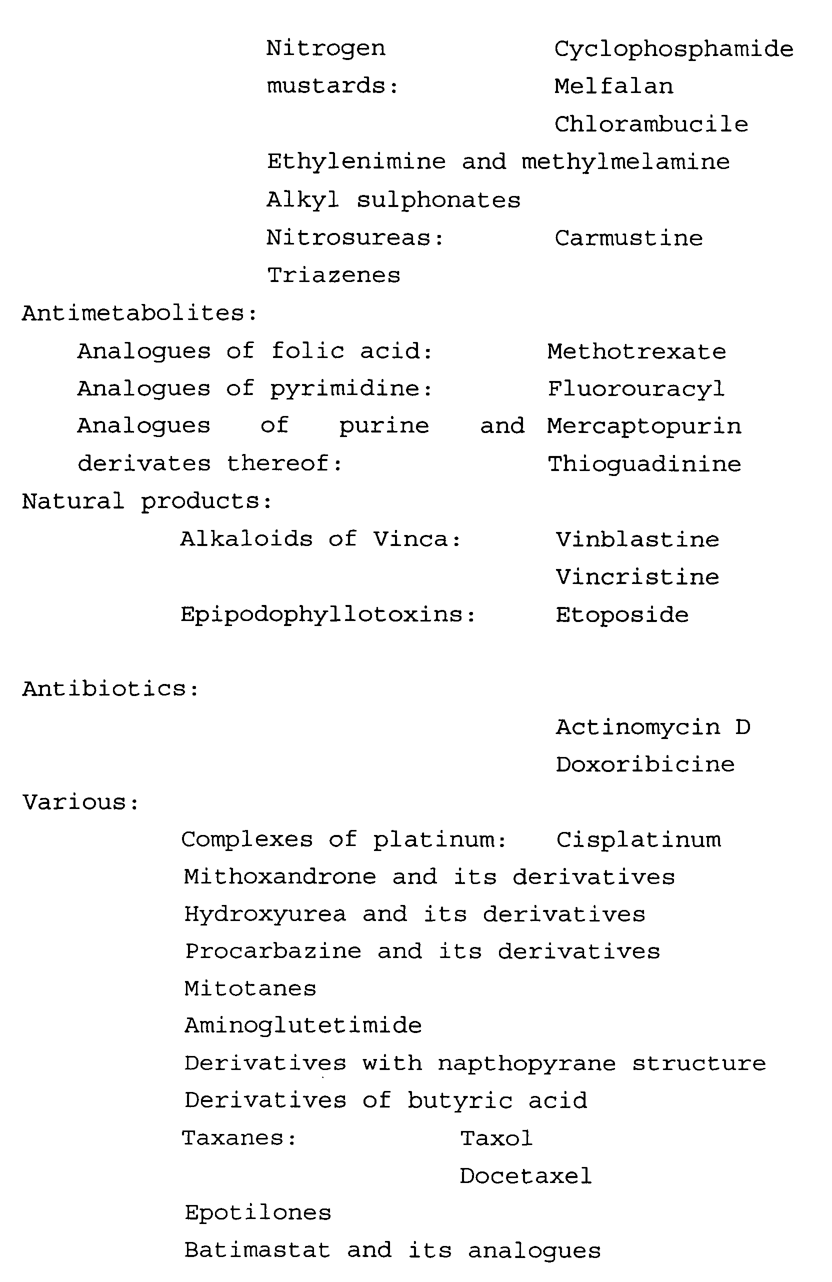

In addition or as an alternative, the active agent or principle may comprise a drug or a complex of drugs with an action that promotes processes of repair of the vessel wall, such as endothelial/angiogenic growth factors; e.g., VEGF or antisense oligonucleotides.

In addition or as an alternative, the active agent or principle may comprise a drug or a complex of drugs that are able to mitigate or prevent the reactions lying at the root of the process of restenosis of a vessel that has undergone stent implantation, such as:

| | |
|---|---|
| Rapamycin | Heparin and the like |
| Actinomycin D | Batimastat |
| Paclitaxel | Resten-NG |
| Dexamethasone | (oligonucleotide) |

Other active principles or agents that can be used in the framework of the present invention are, for example (it is recalled once again that the ensuing list must not be interpreted as in any way limiting the scope of the invention), the ones listed below:

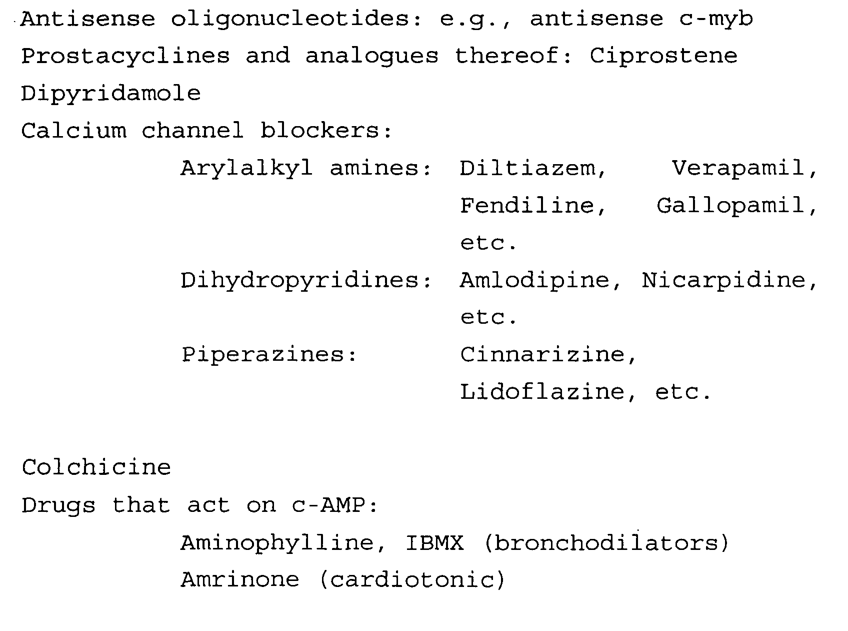

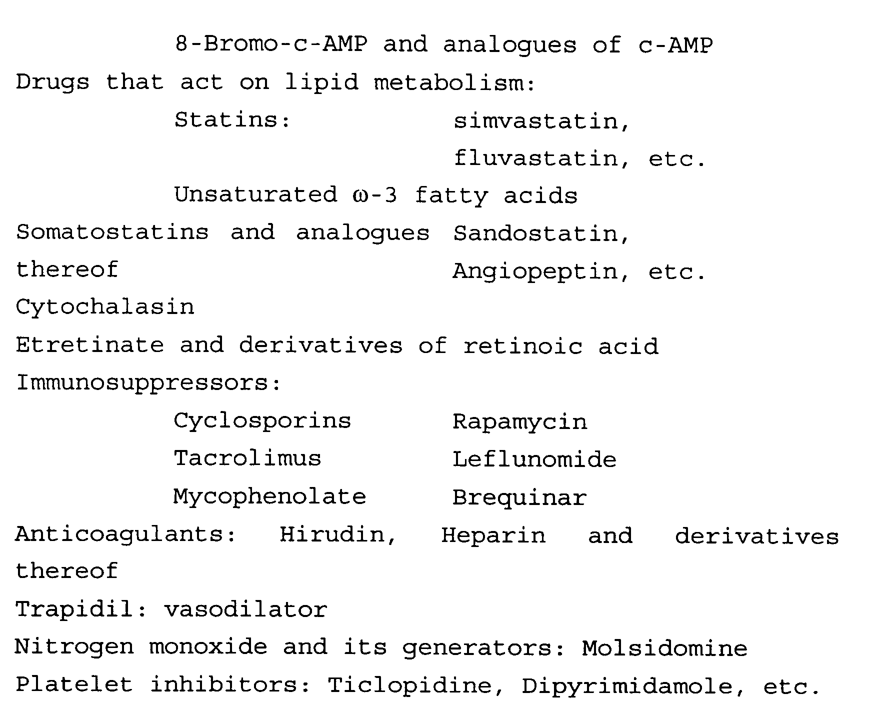

Agents that may act on the activity of the cell and on the regulation of the cell matrix:
proteins (elafin)
oligonucleotides
genes
RNA, DNA and fragments thereof
RNA, DNA and antisense fragments thereof
monoclonal antibodies

Before passing on to a more detailed illustration of the characteristics of the envelope 1b of the nanoparticles 1, useful reference may be made to the general scheme of Figure 2. In this figure, the reference number 2 designates one part of the structure of a stent of any known type, comprising a tubular body (not illustrated as a whole) which is radially expandable and is formed by elements or "struts" that define a reticular structure. The stent may be, for example, of the type illustrated in the document EP-A-0 875 215.

The aforesaid stent generally comprises a plurality of annular elements having a roughly cylindrical shape and a serpentine pattern, which are designed to be aligned in sequence along the main axis of the stent. In the above structure (which is to be held altogether known), the various annular elements are connected together by means of longitudinal connection elements, generally referred to as "links", and have, in the example of embodiment illustrated in the document EP-A-0 875 215, a general lambda conformation. Preferably, the aforesaid connection elements 3 are connected to the cylindrical elements of the stent at the "0" points of the respective sinusoidal paths.

In any case, the geometrical details of the stent do not constitute a limiting or binding element of the invention; the solution according to the invention can, in fact, be applied to stents of any type, shape or size. Even though the invention has been developed with particular attention paid to its possible use in the sphere of stents obtained starting from a microtube, the solution according to the invention can also be applied to stents obtained, for instance, starting from variously shaped filiform materials (the so-called "wire stems").

More in general, it is recalled once again that the solution according to the invention can in general be used together with any carrier that is designed to be placed in an intraluminal position.

According to the general solution proposed in EP-A-0 850 604 (Figures 6 and 7) and developed in the European patent 01830489.9, the elements 2 of the stent, which have in general a filiform or bar-like configuration, are provided, preferably on the surface of the stent facing outwards, with recesses, designated as a whole by 4.

The recesses in question may either basically amount to a single recess which extends, practically without any discontinuities, over the entire development of the stent, or be chiefly, if not exclusively, made in areas corresponding to the rectilinear, or substantially rectilinear, portions of the branches of the stent, thus avoiding in particular both the curved parts (for example, the cusp or loop parts of the elements in question) and the areas in which the connection elements or links are connected to the various annular elements that make up the stent. In particular, formation of the aforesaid recesses may be limited just to the areas of the elements of the stent that will be less subject to stress during operation of the stent.

Again, the recesses 4 may be made in the form of separate wells set at a distance apart from one another and variously distributed over the surface of the stent.

The characteristics of implementation of the recesses described above may, of course, also be used in combination with one another. Consequently, it is possible to have, in one and the same stent, both recesses that extend practically without any discontinuities over an entire portion of the stent and recesses consisting of slits or wells.

However made, the recesses in question are such as to constitute hollowed-out formations which can function as reservoirs to enable arrangement of active/activatable agents, possibly of different types, on the stent.

For example, in the case where recesses 4 are used that have a general well-like conformation, each of the wells constitutes a recess for receiving within it an active/activatable agent having different characteristics. The foregoing affords the possibility of having available on the stent - at least virtually or in principle - as many different agents as there are recesses.

Leaving to one side the above basic hypothesis, the recesses can be used to accommodate different agents in different areas of the stent. For instance, the recesses located at the ends of the stent can receive anti-inflammatory agents since the end parts of the stent are the ones most exposed to the possible onset of inflammatory phenomena; this means that at least one first agent with anti-inflammatory characteristics is present in a higher concentration at the ends of the stent as compared to the central area of the stent. The possibility may then be envisaged of distributing another agent, such as an anti-mitotic agent, with a level of concentration that is constant throughout the longitudinal development of the stent, with the added possibility of distributing yet another agent, such as a cytotoxic or cytostatic agent, with a maximum level of concentration in the central area of the stent and levels of concentration that progressively decrease towards the ends of the stent.

Irrespective of the modalities of construction of the recesses 4 (which in themselves do not form a specific subject of the present invention), it may immediately be realized that the presence of the recesses 4, preferably made on the outer surface of the stent, makes available a wide reservoir for gathering active/activatable agents that can be released from the stent towards the adjacent tissue, which, in the case in point, is exemplified above all in the form of the endothelium E and of the cells C of the smooth muscle.

Since the recesses 4 are made preferably in the outer surface of the stent, the phenomenon of release takes place preferably in a centrifugal direction, i.e., from the outside of the stent 1 towards the wall of the vessel undergoing treatment.

The modalities of construction of the recesses 4 herein illustrated thus make it possible to contain to a very marked extent the phenomena of possible diffusion in a radial direction towards the inside of the stent 1. In this way, it is possible to prevent undesired antagonistic phenomena in regard to the possible neointimal formation.

Again, the fact of having available recesses 4 of large dimensions renders less critical the aspect linked to the physical anchorage of the agent or agents to the surface of the stent. This aspect is particularly important in so far as it makes it possible to apply on the surface of the stent (with the possible exclusion of the surface of the recesses 4, even though this fact is not of particularly determining importance) a layer of biocompatible carbon material (not specifically illustrated in the drawings). This may be, for example, a coating of the type described in the documents US-A-5 084 151, US-A-5 133845, US-A-5 370 684, US-A-5 387 247 and US-A-5 423 886. A coating of carbon material of this sort performs an anti-thrombogenic function, favouring endothelialization and, a factor that is deemed of particular importance, acting in the direction of preventing release of metal ions from the stent 1 to the surrounding tissue.

According to an important characteristic of the invention, the active agents that it is desired to deliver starting from the stent (which acts in more general terms as the body of the carrier) are transported by means of the nanoparticles 1 according to modalities which will be described in greater detail hereinafter.

In particular, it is envisaged that the material of the envelope 1b should be chosen in such a way as to present specific characteristics of selective affinity in regard to organs (or more in general, tissues or regions) that act as targets, the aim being that the nanoparticles, and hence the active principles carried thereby, should concentrate in a selective, and hence differentiated, way in the target regions. In practice, the nanoparticles 1 behave as if they were provided with a sort of driving force that guides them to the target region.

It is thus possible to give rise to a delivery system, which, precisely on account of its selectivity, presents a very high efficiency, with a consequent reduction in the absolute amount of active principle that is to be administered, and hence to be transported by means of the carrier (represented, in the example herein illustrated, by the stent).

In the present case, the target region or regions consist of different types of tissue according to the illness that is to be treated. For example, when a restenosis-antagonistic function is to be performed, the target region is chiefly represented by the cells C of the smooth muscle that surrounds the endothelium E of the vessel.

Consequently, the solution described has a degree of efficiency - and hence a precision of treatment, also as regards local diffusion of the active agent exclusively towards the organs that are to be treated - which is considerably higher than that of traditional solutions. In the traditional solutions in question, the active principle(for example, rapamycin in the case of a restenosis-antagonistic cytostatic function) is released by diffusion, from polymeric matrices arranged on the stent, throughout the environment (blood, first of all, and then plaque and vessel) that surrounds the stent.

Preferably, the envelope 1b of at least some of the nanoparticles 1 is made of a bio-erodible material and/or a material permeable to the active principle that constitutes the core 1a of the respective nanoparticle. Yet again, the envelope 1b of at least some of the nanoparticles may present a stratified structure.

Of course, the representation of Figure 2, in which nanoparticles 1 may be seen that are arranged in such a way as to constitute a mere filling of the recess 4 is to be held purely an example. In particular, the aim of Figure 2 is to illustrate the mechanism of action of the nanoparticles; see in particular the nanoparticles illustrated already in the position of migration through the endothelium E and inside one of he cells C.

By way of example, assume that the aim is to transport to the cells C an active principle, such as an immunosuppressor like rapamycin, at the same time containing and virtually preventing transport of the said agent towards and within the endothelium E. In this case, the active principle is included in the cores 1a of the nanoparticles 1, and in the envelopes 1b of the nanoparticles 1 themselves there are instead provided functional groups of recognition of the muscle cells C, such as peptide sequences or proteins of recognition (antibodies) or fractions/fragments thereof. A specific example in this connection is represented by the sequences of the type arginine-glycine-aspartic acid (RGD).

The above mechanism of selective delivery/diffusion of the active principle to the cells C is therefore linked to the fact that the nanoparticles are provided with envelopes 1b having differentiated characteristics of affinity attraction in regard to the various regions (hence to the various organs) corresponding to the site of implantation of the carrier.

When the carrier is located in the site of implantation, each nanoparticle migrates primarily and selectively towards a region (namely, towards an organ) in regard to which the nanoparticle has greater affinity attraction, thus giving rise to a selective mechanism of delivery of the active principle or active principles carried thereby.

The above characteristic can be exploited for providing, in the recesses 4 of the carrier, both fillings of nanoparticles of a homogeneous type and fillings of nanoparticles comprising nanoparticles of at least one first species and one second species, which are different from one another.

For example, assume that (in addition to selectively delivering rapamycin to the cells C) the aim is to deliver to the endothelium E an agent (for example, VEGF) aimed at favouring re-growth of the intima of the endothelium E itself, at the same time preventing (or at least containing) delivery/diffusion of said active principle to the cells C.

In this case, in addition to the nanoparticles 1 seen previously, it is possible to envisage the presence, in the recess or recesses 4, of a second species of nanoparticles 1, the cores 1a of which transport the agent VEGF, whilst the corresponding envelopes 1b are substantially of a lipidic nature, consisting, for example, of stearic acid.

There is thus obtained a preferential, and hence selective, administration of the agent VEGF in the endothelium E (and in particular in the first layers facing the stent), at the same time obtaining preferential and selective delivery of rapamycin to the cells C.

In a complementary way, it is possible to provide, on the carrier, recesses 4 each of which is designed to receive a filling of nanoparticles which are all of the same species, the various recesses containing nanoparticles of different species.

The aforesaid mechanisms of differentiation of the species of nanoparticles within the individual recess or in the framework of different recesses can be used in a combined way, in particular in different regions of the stent, if necessary again exploiting other factors, such as the possibility of dispersing the active principles within polymeric matrices, in particular of a bio-erodible type.

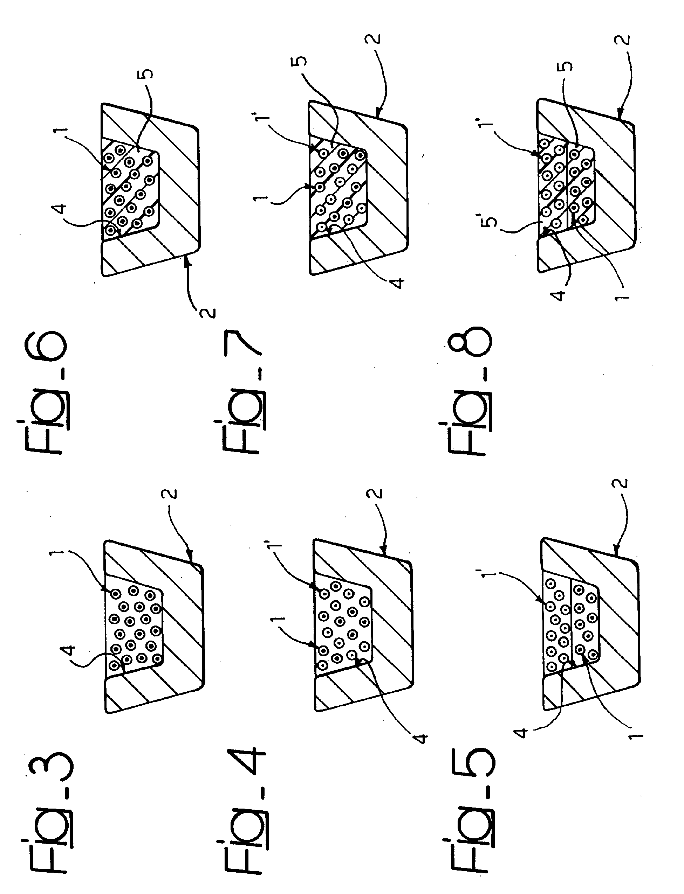

The flexibility of the corresponding mechanism is illustrated, purely by way of example, in Figures 3 to 9.

In particular, Figure 3 basically re-proposes, in a schematic way, the solution of Figure 2, with the nanoparticles 1 constituting a filling directly contained in the recess 4 of the carrier.

Figure 4 relates, instead, to a solution in which in the recess 4 there are present two different species of nanoparticles, one of which is designated by 1 and the other by 1'.

The two species are differentiated in at least one of the characteristics typical of the core 1a and/or of the envelope 1b such as, for example, at least one of the following characteristics:
- bio-erodible nature of the envelope 1b;
- time of erosion of the envelope 1b
- permeability of the envelope 1b to the active principle contained in the respective core 1a;
- thickness of the envelope 1b;
- stratified structure of the envelope 1b and
- characteristics of selective affinity attraction of the material constituting the envelope 1b in regards to said at least one first region and one second region.

The two species of nanoparticles 1 and 1' are mixed together and again constitute a free filling of the recess 4.

In the example of Figure 5, there are again present two species of nanoparticles 1, 1' which are different from one another. However, instead of being mixed together as in the case of the solution of Figure 4, in the solution of Figure 5 the nanoparticles in question form two layers 1, 1' set on top of one another. The solution of Figure 5 is evidently designed to be preferably used in those applicational contexts in which the aim is that the active principle conveyed by the nanoparticles 1' should be delivered prior to the active principle conveyed by the nanoparticles 1.

The solutions illustrated in Figures 6 to 8 essentially correspond to the same solutions as those illustrated in Figures 3 to 5, respectively, with the difference that, in the case of the solutions of Figures 6 to 8, the nanoparticles 1, 1' do not simply constitute a free filling of the respective recess but are instead received in one or more corresponding polymeric matrices 5, 5'.

Figure 9 illustrates yet another possible variant embodiment of the invention.

In this solution, inside the recess 4 there are arranged, starting from the bottom of the recess 4, the following:
- a layer of active principle (for example, a drug 6) set in a respective polymeric matrix;
- a layer comprising two species of nanoparticles 1, 1' which are mixed together (and are possibly incorporated in a respective polymeric matrix); and
- a top layer 7 of bio-erodible polymeric material which closes, in the manner of an operculum, the top aperture of the recess 4.

The presence of the operculum of polymeric material 7 is evidently designed to cause delivery of the active principles present in the underlying recess 4 to start only after the aforesaid operculum layer 7 has been eroded and/or rendered permeable in regard to said active principles.

The stent acting as a carrier body can therefore comprise a plurality of recesses 4 that have the function of reservoirs, the said plurality comprising at least one first recess 4 and at least one second recess 4 which have associated thereto respective masses of polymeric material which are differentiated from one another in at least one characteristic chosen in the group made up of:
- function of the polymeric mass as a matrix or as a closing operculum of the reservoir;
- bio-erodibility of the polymeric mass;
- time of erosion of the polymeric mass;
- permeability of the polymeric mass to the active principle or principles conveyed by the nanoparticles;
- thickness of the polymeric mass; and
- stratified structure of the polymeric mass.

As regards the characteristics of the recesses 4, the delivery mechanism described can draw considerable advantage in terms of flexibility from the possibility of intervening selectively on parameters such as:
- size and shape of the individual recess;
- location of the recess on the carrier body;
- blind or through character of the recess.

In a particularly preferred way, the carrier has surfaces, an outer one and an inner one, with respect to the site of intraluminal implantation, and the recesses are located on the outer surface.

Of course, without prejudice to the principle of the invention, the details of construction and the embodiments may vary widely with respect to what is described and illustrated herein, without thereby departing from the scope of the present invention as defined in the claims which follow.

## Claims

1. A carrier for delivering at least one active principle at an intraluminal site comprising at least one first region (C) and at least one second region (E), **characterized in that** it comprises:
- a carrier body (2) which can be conveyed to said intraluminal site and is provided with at least one reservoir (4) for receiving said at least one active principle; and
- in said at least one receiving reservoir, a filling of nanoparticles (1, 1'), said nanoparticles conveying (1a) said at least one active principle and comprising (1b) at least one substance having characteristics of affinity of preferential attraction in regard to a preferred region between said at least one first region and said at least one second region, whereby, with said carrier body (2) located in said intraluminal site, each of said nanoparticles mainly migrates towards said preferred region in regard to which said at least one substance has characteristics of preferential affinity attraction.

2. The carrier according to Claim 1, **characterized in that**, in said nanoparticles (1, 1'), said at least one substance (1b) is at least partially distinct from said at least one active principle (1a).

3. The carrier according to Claim 1 or Claim 2, **characterized in that** said nanoparticles (1, 1') comprise a core (1a) that conveys said at least one active principle, and an envelope (1b) with associated said at least one substance presenting characteristics of preferential affinity attraction.

4. The carrier according to Claim 3, **characterized in that** the envelope (1b) of at least some of said nanoparticles is made of bio-erodible material.

5. The carrier according to Claim 3 or Claim 4, **characterized in that** the material of the envelope (1b) of at least some of said nanoparticles is permeable to the active principle constituting the core (1a) of the respective nanoparticle.

6. The carrier according to any one of Claims 3 to 5, **characterized in that** the envelope (1b) of at least some of said nanoparticles has a stratified structure.

7. The carrier according to any one of the preceding claims, **characterized in that** said filling of nanoparticles comprises nanoparticles of at least one first species (1) and one second species (1'), said two species of nanoparticles being differentiated from one another in at least one characteristics of said at least one active principle (1a) and/or of said at least one substance (1b).

8. The carrier according to Claim 3 and Claim 7, **characterized in that** the nanoparticles of said first species (1) and said second species (1') are distinguished from one another in at least one of the following characteristics:
- bio-erodible nature of the envelope (1b);
- time of erosion of said envelope (1b);
- permeability of said envelope (1b) to the active principle contained in the respective core (1a);
- thickness of the envelope (1b);
- stratified structure of the envelope (1b); and
- characteristics of selective affinity attraction of the material constituting the envelope (1b) in regards to said at least one first region and one second region.

9. The carrier according to Claim 1 or Claim 7, **characterized in that** said carrier body comprises a plurality of said reservoirs with respective fillings of nanoparticles at least partially of different species (1, 1').

10. The carrier according to any one of the preceding claims, **characterized in that** said at least one active principle is chosen in the group consisting of anti-inflammatory agents, antineoplastic agents, vessel-wall repair agents, and restenosis-antagonist agents.

11. The carrier according to any one of the preceding claims, **characterized in that** said at least one substance is chosen in the group consisting of functional groups of recognition of muscle cells, peptide sequences or proteins of recognition, antibodies and fractions/fragments thereof.

12. The carrier according to any one of the preceding claims from Claim 1 to Claim 11, **characterized in that** said at least one substance comprises a sequence of the arginine-glycine-aspartic acid (RGD) type.

13. The carrier according to Claim 10, **characterized in that** said at least one active principle is an agent, such as VEGF, for favouring re-growth of the intima of the endothelium (E).

14. The carrier according to Claim 1 or Claim 13, **characterized in that** said at least one substance is a substance of a lipidic nature, such as stearic acid.

15. The carrier according to any one of the preceding claims, **characterized in that** at least one part of said filling of nanoparticles (1, 1') carries, associated thereto, a mass of polymeric material (5, 5', 7) having the function of a dispersion matrix or else of a closing operculum of the respective reservoir.

16. The carrier according to Claim 16, **characterized in that** it comprises a plurality of said reservoirs (4), said plurality comprising at least one first reservoir and at least one second reservoir which have associated thereto respective masses of polymeric material (5, 5', 7) that are differentiated from one another in at least one characteristic chosen in the group made up of:
- function of the polymeric mass as a matrix or as a closing operculum of the reservoir;
- bio-erodibility of the polymeric mass;
- time of erosion of the polymeric mass;
- permeability of the polymeric mass to said at least one active principle;
- thickness of the polymeric mass; and
- stratified structure of the polymeric mass.

17. The carrier according to any one of the preceding claims, **characterized in that** said at least one reservoir is defined by a recess (4) provided on the surface of said carrier.

18. The carrier according to Claim 17, **characterized in that** it comprises a plurality of said recesses, said plurality comprising at least one first recess (4) and at least one second recess (4) that are differentiated from one another in at least one characteristic chosen in the group made up of:
- size of the recess;
- shape of the recess;
- location of the recess on said carrier body; and
- blind or through character of the recess.

19. The carrier according to any one of the preceding claims, **characterized in that** said carrier (2) has surfaces, namely an outer surface and an inner surface, with respect to said intraluminal implantation site, and **in that** said at least one reservoir (4) is located on said outer surface.

20. The carrier according to any one of the preceding claims, **characterized in that** said carrier body is configured in the form of a stent comprising a radially expandable tubular body formed by elements defining a reticular structure, said at least one reservoir (4) being provided in a respective element (2) of said reticular structure.

21. A kit for delivering at least one active principle in an intraluminal site, **characterized in that** it comprises:
- a carrier according to any one of Claims 1 to 20; and
- an inserter means for placing said carrier in an intraluminal site.

22. The kit according to Claim 21, **characterized in that** said inserter means is a catheter.

23. The kit according to Claim 22, **characterized in that** said catheter is a balloon catheter.
